# EUROPEAN PATENT SPECIFICATION

(11) **EP 0 774 116 B1**
(45) Date of publication and mention of the grant of the patent: **03.11.1999**
(21) Application number: 95925915.1
(22) Date of filing: 19.07.1995
(51) Int. Cl.: G01N 33/50, G01N 33/543, G01N 33/58

(54) **METHOD FOR SCREENING COMPOUND LIBRARIES**
VERFAHREN ZUR SICHTUNG EINER BIBLIOTHEK VON CHEMISCHEN VERBINDUNGEN
PROCEDE DE CRIBLAGE DE BANQUES DE COMPOSES

(30) Priority: 22.07.1994 GB 9414770; 19.05.1995 GB 9510137
(43) Date of publication of application: 21.05.1997
(73) Proprietor: ZENECA LIMITED, London W1Y 6LN (GB)
(72) Inventor: GARMAN, Andrew, John, Chester CH3 8DE (GB); HOLLAND, Janet, Dora, Poynton Cheshire SK12 1DZ (GB)
(74) Representative: Giles, David Eric
(86) International application number: GB9501700
(87) International publication number: WO9603647

(56) References cited:
- WO-A-93/24517
- WO-A-94/02515
- WO-A-94/08051
- TETRAHEDRON LETTERS, vol. 31, no. 45, 29 October 1990 OXFORD GB, pages 6493-6496, G. T. WANG ET AL. 'Design and synthesis of new fluorogenic HIV protease substrates based on resonance energy transfer.'
- CLINICAL CHEMISTRY, vol. 39, no. 9, September 1993 WINSTON US, pages 1953-1959, G. MATHIS. 'Rare earth cryptates and homogenous fluoroimmunoassays with human sera.' cited in the application

## Description

The production of combinatorial compound libraries on beads is a powerful way of synthesising large numbers of relatively diverse new chemical entities. The screening of compounds produced by such strategies is however not straightforward, particularly when the compounds so produced are not peptides. A review of published approaches may be found for example in J. Med Chem. (1994) 37, 1233. Combinatorial approaches are a powerful way of identifying novel leads in the pharmaceutical and agrochemical industries.

Any novel testing approach should preferably fulfill the following criteria:
1. The compound should be tested in free solution, not bound to a solid phase.
2. There should be no necessity to employ a purified protein target, i.e. the method should be capable of working with unpurified preparations of biological targets of interest.
3. There should be a level of specificity in the assay, such as is provided by a competitive system where the test compound is required to compete with a ligand for a receptor, or specifically inhibit an enzyme.
4. The compound should be present at a high concentration, for example above 10 uM, preferably above 100uM. Such concentrations are not readily achieved when libraries are tested as mixtures.

The present invention now provides novel methods which meet the above criteria. They may be used for the screening of compound libraries. They employ a technique which we refer to as zone screening. Compounds are released from a solid phase into distinct zones of an assay medium, which medium is capable of reporting distinct zones of activity.

Therefore in a first aspect of the present invention we provide a method for screening a compound library provided on solid phase, which method comprises releasing a proportion of the library from the solid phase into distinct zones of an assay medium, performing a proximity screening assay with a biological of interest within the medium, identifying active zone(s) in the assay medium and determining the identity of active member(s) of the library by reference to the corresponding compound(s) still bound to the solid phase.

By "proximity screening assay" we mean an assay where a desired interaction, cooperation or binding between two species is detected by a signal that depends on the proximity of a first signalling component with a second signalling component. Signalling components may be extrinsic signals such as radiolabels, fluorophores, chemilumophores and scintillants or intrinsic signals such as the mass or chemical environment of an assay component.

The use of proximity assays is not foreshadowed in the art and offers a number of significant advantages. There are no separation steps involved which would be cumbersome. Also, proximity assays give a signal in real time, or close to real time, which may provide useful kinetic information. Real time also gives flexibility in not having to stop an assay at a given time, thereby allowing both very weak actives and very strong actives to be determined in the same experiment.

The proximity screening assay may be a fluorescent assay such as assays based on fluorescence resonance energy transfer (FRET), fluorescence polarization, fluorescence correlation microscopy, fluorescent probes or other related fluorescent assay principles. Such assay principles can be employed to design assays that are able to detect compounds that inhibit a range of systems of pharmacological interest, such systems including enzymes, receptor interactions and protein-protein interactions [see for example Tetrahedron letters. 31, p. 6493-6496, G. T. WANG et al.

A preferred fluorescence-based proximity assay is fluorescence resonance energy transfer. Such assays are based upon the bringing together or pulling apart of two labelled species. Thus in receptor-ligand binding assays or protein-protein interaction assays, where it is desired to find an inhibitor of the binding or interaction, one component is labelled with a donor fluorophore and the second component is labelled with an acceptor. When the two components are together, energy transfer is observed which may be made manifest either by a quenching of the donor fluorescence or (if the acceptor has the suitable fluorescence characteristics) by an increase in the fluorescence of the acceptor. An active compound is then revealed by a diminution in the FRET observed.

FRET may also be used to detect inhibitors of certain enzymes, for example proteases. Preferred methods for synthesising protease FRET substrates have been described, see for example our WO-94/28166 (Zeneca). Alternatively, binding substances, for example antibodies, that bind specifically to the product of an enzyme reaction may be employed and assays designed that are based on for example FRET occurring between a donor-labelled product and an acceptor-labelled binding substance (or vice versa). This latter approach may conveniently be used for example for protein kinases where the substrate is a donor-labelled peptide.

By FRET we also mean energy transfer assays employing long lived donors for example certain lanthanide chelates and cryptates. Such assays have been described in the literature; see for example Matthis, G., Clin. Chem (1993), 39, 1953-1959.

preferred proximity assays include assays based on the proximity of a radiolabel to a scintillant. Here a support comprises a scintillant and a biological of interest. The support is preferably of a plastic such as polystyrene. The biological of interest is conveniently a receptor, optionally a membrane receptor contained in membrane fragments, or a target protein, or a member of a specific binding pair able to capture an assay component labelled with the complementary pair. Suitable specific binding pairs include avidin (or streptavidin) and biotin, or antibody and antigen (or hapten). Lectins are also convenient binding components and may be used with suitable glyocoproteins, cells or cell membrane fragments. The biological of interest is coated on to the scintillant-containing support, for example by passive coating. Optionally, agents that promote binding to the support may be employed. We have found that pre-treatment of the support with certain positively charged compounds which have an affinity for plastics such as polystyrene is beneficial in coating cell membrane fragments. Polyethylene imine is a preferred coating agent.

In the screen the biological of interest captures a radiolabelled compound, the radiolabel being chosen from those that show a proximity effect as described by Hart and Greenwald (Mol. Immunol. 16, 265-267 (1979)). Conveniently the radiolabel is selected from 125-I, 3-H or 33-P. Active compounds released from the beads inhibit binding of the label to the biological of interest. Only radiolabel captured by the biological of interest causes light emission from the scintillant. Active compounds are therefore indicated by a "dark" zone where light emission is reduced.

In a related approach, beads containing scintillant and coated with the biological of interest are incorporated into the assay medium. Such beads have been described, see for example Udenfriend et al., Anal. Biochem. 161, 494-500 (1987). This approach is referred to as the scintillation proximity assay (SPA, Amersham).

Proximity assays also include those based on fluorescence energy transfer (FRET) where the acceptor fluorophore is immobilsed either on or in the support, or is attached to the biological of interest coated onto the support. In the case of assays involving an immobilised receptor, the released test compound competes with a ligand for the receptor which has been labelled with a FRET acceptor. In the absence of an active compound the labelled ligand binds to the immobilised receptor and FRET is observed. Active compounds are revealed by zones of reduced FRET. It will be appreciated that this principle may be used for other assays, for example enzyme assays, where for example, a labelled enzyme product binds to a binding partner on the solid phase. In some cases, it may be advantageous to interchange donor and acceptor.

Alternatively, the proximity assays of the invention may be used to measure or detect a biological of interest, for example secreted by a cell or produced by enzyme action. This may conveniently be achieved for example by an immunoassay including analogous procedures. In the scintillation proximity assay approaches, an antibody to the biological of interest is attached to the solid phase. This captures the biological of interest which is revealed by a labelled second antibody which forms a sandwich. Competitive assays and other variations will be apparent to the scientist of average skill. Likewise fluorescence proximity assays, for example FRET assays, may be devised; see for example the FRET immunoassay reference by Matthis cited above. By these approaches compounds may be detected that alter the level of the biological of interest. Alternatively, bound fluorescently labelled species which act as an end-point for the assay may be selectively excited using evanescent wave technology.

The compound library may comprise a chemical compound library or a bio-oligomer library.

By bio-oligomers we mean compounds synthesised by the addition of multiple building blocks in a linear fashion, such building blocks being those used in nature, for example amino acids or nucleotides, or analogues thereof. Bio-oligomers includes, for example, peptides.

By chemical compound library we mean a library comprising any convenient number of diverse non-oligomeric compounds synthesised from different types of building block precursors. Particular chemical library compounds are of low molecular weight and potential therapeutic agents. They are for example of less than about 1000 daltons, such as less than 800, 600 or 400 daltons. They include for example template-based smaller molecules, for example benzodiazepines, hydantoins, biaryls, polycyclic compounds (eg. naphthalenes, phenothiazines, acridines, steroids etc.), carbohydrate and amino acid derivatives, dihydropyridines, benzhydryls and heterocycles (eg. triazines, indoles etc.). The numbers quoted and the types of compounds listed are illustrative, but not limiting. Chemical compound libraries may be assembled by a number of methods, including the 'combine/mix/divide' process described by Furka et al (Abstr. 14th Int.Congr.Biochem., Prague, Czechoslovakia, 1988, 5, 47; Int.J.Pept.Prot.Res, 1991, 37, 487-493) for creating libraries on polymer beads, in which each bead contains one discrete chemical species. Chemical libraries may also contain tags such as is disclosed in WO 94/08051.

The compound library is preferably synthesised in a combinatorial fashion.

By "solid phase" we mean either a single solid phase comprising distinct regions such as an array of pins or we mean a plurality of individual solid supports such as beads. By the latter we mean spheroidal, preferably porous particulates or any other convenient particulate phase which allows dispersal into the assay medium and which allows a proportion of the library to be released. Conveniently, the beads are of derivatised polystyrene or polyacrylamide as described by example E. Bayer in Ang. Chem. Int. Ed: 30, 113-216 (1991). Optionally, they may have hydrophilic spacer groups such as poly- or oligo- ethylene glycol.

By "pins" we mean formations made of a convenient material, for example plastic such as polystyrene or polypropylene, optionally treated or formed to increase their surface area, a plurality of which forms an array. Compounds may be synthesised on pins. Alternatively, the pins may be used to transfer compounds, for example in solution, from a stock vessel (or array of vessels, for example a microtitre plate) to the assay medium. Transfer pins may optionally be made from other inert materials for example stainless steel.

Optionally the beads have a common visible tag, for example a coloured tag or a fluorophore emitting in the visible part of the spectrum, in order to facilitate the location, removal and/or handling of the bead after the assay.

Compounds are released for example by hydrolysis of a linker or by the cleavage of a photo-labile linker with light. The release may be effected either before, during and/or after the beads have contacted the assay medium. Such linkers are described for example by Albericio et al:Int J Pept Prot Res: 30,106-216(1987), and Pillai, Synthesis:1-26(1980) or will be readily apparent to a chemist of ordinary skill. If release is effected before dispersal in the assay medium then preferably the dispersal should be implemented sufficiently quickly such that leakage of compound from one bead to an adjacent bead should not occur to any deleterious extent. Alternatively beads may be spaced out during the cleavage reaction. Release before contacting the assay medium may be effected by a wider range of methods appropriate to the chemical strategy that is used to prepare the compounds. Thus for example agents such as acids, bases and fluoride ions may be employed. Particulary preferred agents are trifluoroacetic acid, volatile primary and secondary amines, and tetramethylammonium fluoride. The agents chosen should preferably be volatile or able to be neutralised in a rapid fashion for the reasons outlined above.

It will be appreciated that protecting groups may be employed in the synthesis of the library and that these may be selected to be compatible with the cleavage strategy employed. After synthesis any protecting groups on the compounds may be removed by a method which does not result in cleavage of the compound from the support. For example, if the compounds are linked to the support via a base labile linker the protecting groups may be removed by treatment with TFA. The compounds are then released for example by ammonolysis.

The proportion of the library to be released depends on the desired level of activity that it is desired to detect and the method for identifying the compound that gives the activity. Sufficient must be released to give detectable activity (if present), while sufficient must be retained to allow identification. In general for compounds synthesised on beads and with loadings currently possible, release of up to 10% of the compound may give sufficient local concentrations for testing purposes, whilst approaching 100% release may still result in some released material being retained by the bead. This material may be sufficient in most cases to detect by for example mass spectrometry. The proportion of the library to be released may therefore be determined by the scientist of ordinary skill and will normally be in the range 1% to 100%, preferably 10-90%.

The assay medium is selected such that diffusion of the relevant reporter assay components can take place at a rate that is sufficiently fast to allow these to interact in a convenient time period, but not so fast that equilibrium is achieved before sufficient library compound can diffuse from the solid support to interact with the reporter assay system. The assay medium conveniently comprises a physical support, such as a sheet of plastic such as polystyrene, having an optional boundary region, containing or supporting a gel phase. In which case the components of the assay may be contained in the gel phase and, optionally, coated onto the support. The gel may be any convenient substantially aqueous gel for example agar, agarose, polyacrylamide or beaded gels such as Sepharose (Pharmacia) or a matrix, for example a fibrous matrix such as glass fibre or paper. With respect to the issue of diffusion rates referred to above, it will be appreciated that the porosity of the gel phase needs to be taken into account. Selection of a gel phase with the appropriate properties for a given assay may be achieved by routine experimentation and without undue effort or inventive skill.

Where the solid phase is beads these may be dispersed in the assay medium by random or non-random methods. Random methods include adding the beads, optionally in suspension form, to the assay medium prior to casting, mixing thoroughly and casting the gel phase. This is the preferred method. Assay components may optionally be added prior to casting. Optionally beads may be added in a random fashion after the casting the gel but before it sets. This may be achieved by adding to the level gel a suspension of the beads in a suitable liquid. Optionally, beads may be added in a random fashion after the gel has set. Alternatively the beads may be added in a non-random fashion, for example in a regular array, using a suitable bead dispensing device, or by application of a sheet with the beads attached. In this case beads are added after casting but before setting, or alternatively after setting.

By "casting" we mean the formation of even spread of the gel phase using methods appropriate to the nature of the gel. For example, an agarose gel is formed by first melting the agarose, casting and allowing the agarose to set. Alternatively this may involve the pouring of a suspension or slurry onto a level surface to form a layer and allowing the gel to form. The thickness of the layer is preferably in the range 0.lmm to 5mm such as 0.1mm to 2mm, more preferably 0.2mm to 2mm such as 0.2mm to 1mm.

By "bound to the solid phase", we mean either covalently or non-covalently attached to the solid phase, or associated with the solid phase. This association may either be direct, that is selection or removal of the solid phase selects also the compound for identification, or indirect. By way of example an identifier on the solid phase enables the operator to locate and identify the compound. The identifier may be for example the position of the solid phase in an array or grid.

Significantly zone screening approaches have hitherto only been applied to the identification of peptides. We have now determined that zone screening may be successfully applied to chemical compound libraries.

Therefore in a further aspect of the present invention we provide a method for screening a chemical compound library provided on solid phase which method comprises releasing a proportion of the library from the solid phase into distinct zones of an assay medium and performing a screening assay with a biological of interest within the medium, identifying active zone(s) in the assay medium and determining the identity of active member(s) of the library by reference to the corresponding compound(s) still bound to the solid phase.

The chemical compound library is preferably synthesised in a combinatorial fashion and/or the solid phase preferably comprises a plurality of individual solid supports such as beads.

The screening assay to be performed in the assay medium may comprise the growth of organisms, particularly micro-organisms, cell-based reporter gene assays, growth/no-growth assays, a homogeneous assay based on, for example, fluorescence, or a homogeneous colorimetric assay for an enzyme. Convenient micro-organisms include pharmaceutical or agrochemical targets where the object is to identify compounds that will kill, modulate growth or modulate morphology of the micro-organism. Micro-organisms include bacteria, fungi, mycoplasma, parasitic organisms and viruses as provided in a suitable host.

Cell-based assays include assays where the cells used contain naturally, or are engineered by recombinant means to contain, systems which give readouts in response to desired events (Hertzberg, R.P. (1993) Current Opinion Biotechnology 4, 80-84). Such assays include growth/no-growth assays, reporter gene assays and reporter protein assays. In growth/no-growth assays, the desired event either promotes or inhibits the growth of cells. Such assays are conveniently performed with micro-organisms such as the bacterium Esherischia coli or the yeast Saccharomyces cerevisiae. In reporter gene assays, inhibition or activation of transcription of a gene is measured. This gene is conveniently an enzyme such as E. coli beta-galactosidase or firefly luciferase, or some other protein whose quantity may be easily measured, such as green fluorecent protein from the jellyfish Aequorea victoria. Transcription of reporter genes can be made dependent upon a variety of different events, including protein:protein interactions, receptor:ligand interactions, and protein:RNA interactions (Hertzberg, ibid.; Kirsch, D.R. (1993) Current Opinion Biotechnology 4, 543-552). In reporter protein assays, a protein which is sensitive to desired events is expressed in a cell. For example, the fluorescence of the A. victoria protein aequorin may be used to monitor levels of intracellular calcium.

Preferred screening assays include proximity assays as described above.

Active member(s) of the library may be identified by any convenient technique or combination of techniques. These may include for example mass spectrometry, identification of synthesis tags, for example fluorescent or coloured tags or nucleic acid tags, or iterative synthesis and re-assay.

It will be appreciated that zones of activity may be detected using imaging equipment appropriate to the signal to be detected. Equipment to measure scintillation events, chemiluminescence, colour or fluorescence is available commercially or may be constructed for the purpose. Some coloured or fluorescent signals may be detected directly by the human eye.

Once the zones giving rise to activity have been identified, the beads are physically removed from the gel phase. This may be facilitated by use of the visible tag as described above. Beads may removed by any convenient means, for example using fine tweezers or capillary tubes. Adherent material from the assay medium may be removed for example by manual manipulation or by washing.

It will be appreciated that where the beads have been dispersed by a random method, zones of activity may be associated with more than one bead. Further identification experiments may be performed in order to identify which bead or beads gave rise to the activity. Accordingly, we disclose a method whereby the method of the invention is performed two or more times, first at a higher density of beads, removing the beads from the activity zone or zones, and repeating the method of the invention with those beads spread at a substantially lower density. In this fashion it will be possible to obtain single beads which may be unambiguously identified.

By way of non-limiting illustration, a sheet of plastic, for example polystyrene is coated with a scintillant suitable for scintillation proximity assays. This then is passively coated with membrane fragments containing a target receptor. Optionally this is facilitated by pre-treating the support with poly ethylene-imine.

A gel phase is prepared as follows. To a suspension of or solution of agarose, dextran or acrylamide beads, for example Sepharose (Pharmacia), is added radiolabelled ligand and the beads containing the compounds. The suspension is mixed thoroughly and then poured onto the level support, contained in a suitable tray. The gel is allowed to form and excess fluid removed. Compounds are released for example as they contact the aqueous phase by hydrolysis (cf. Hoffmann et al, Tetrahedron Letters, 1994, 35 (42), 7763-7766) or by irradiating the gel with light of a suitable wavelength. The method used will depend on the chemical design of the library. The gel is then incubated at a suitable temperature, for example room temperature, optionally sealed to prevent further evaporation, for a convenient time period. During this incubation, radiolabelled ligand will bind to the receptor on the support and compounds will diffuse from out of the beads to give a concentration gradient around the bead. Compounds able to bind to the receptor at the ligand-binding site will prevent or reduce the extent of binding of the radiolabelled ligand to the receptor.

The gel is then imaged for example using a sensitive camera-based imaging system. Since the scintillant in scintillation proximity mode reports only ligand bound to the support, active compounds will be revealed by zones of darkness in a light background. The active beads are then identified in the gel by matching an image of the gel with the gel itself, removing the beads of interest, for example using tweezers, briefly washing the beads and identifying the compounds for example by mass spectrometry. The above procedure may also be carried out with SPA beads instead of the scintillant-coated plastic.

WO-94/02515 (Bunsen Rush Laboratories) discloses a method for releasing oligomeric compounds from beads with gaseous reagents and detecting such compound with a melanophore reporter system. Beads are spread on a film and compounds are cleaved with volatile reagents. The film is then contacted with the cells. This is one useful approach that may be adopted in the method of the invention.

For many assay types, we have found that it is advantageous to apply the components of the assay in more than one layer. For example, when seeking inhibitors of an enzyme or a binding interaction, it is generally desirable to contact the test compound with one of the assay components before adding other assay components. Thus, for example, it is desirable for test compounds to contact an enzyme or receptor, before adding the corresponding substrate or ligand, respectively. Thus we disclose a method according to the method of the invention where a first layer containing one or more assay components is formed, the compounds contacted with this layer, and a second layer containing one or more assay components is formed on top of the first.

It will be understood that after the two layers have been contacted with each other, the assay components will diffuse together to give a signal. At or near this interface, compounds will be released from the beads and give zones of inhibition. The nature of the layer is preferably a gel as described above. The layers may or may not be of the same material. Agarose is a preferred material. For reporter gene assays based on an enzyme output, conveniently the substrate is added in a separate layer. Optionally, three layers may be advantageous in certain applications. Optionally, the compounds may be added after the layers have been contacted together.

Alternatively, one layer may contain an assay component in beaded form, and a second layer contain a further soluble assay component. This approach may conveniently be used for example with SPA beads, or any other biological of interest coated on a bead or or equivalent solid phase.

It will be understood that there are alternative ways of delaying the availability of one of the assay components. For example one assay component is delivered by means of a reagent bead, where the assay component is absorbed onto the bead such that, when contacted with the assay medium, it is able to diffuse slowly into the assay medium. Such alternatives are included in the scope of the invention.

The methods of the invention are useful for the identification of new compounds acting at a wide variety of receptor, protein-protein interaction, enzyme and other targets of pharmaceutical or agrochemical importance. They are also useful for research purposes in the identification of compounds interacting with targets of interest for the development of research tools and the identification of new targets of interest.

The advantages of the method of the invention include:
1. Compounds are tested essentially free in solution. This contrasts with other methods where the target of interest is required to bind to the compound on a solid phase. This has the disadvantage of being complicated by steric interference and other problems caused by the solid phase, and by the possibility that the target protein may bind advantitiously by sites on the protein other than the site of interest, leading to false positives.
2. There is no requirement for the target of interest to be in a purified form.
3. Assays may be employed that are competitive in nature, for example where the compound has to compete with the labelled ligand for the receptor. This reduces the incidence of false positives. Specificity is also obtainable by use of functional assays, for example enzyme assays, bacteriacidal assays and certain reporter gene assays.
4. The compounds are tested effectively in isolation rather than as mixtures, as required by some compound library testing strategies. This permits much higher concentrations of individual compounds to be obtained, greatly increasing the chances of finding activity of interest.

The invention will now be illustrated but not limited by reference to the following Examples, Table and Figure wherein:

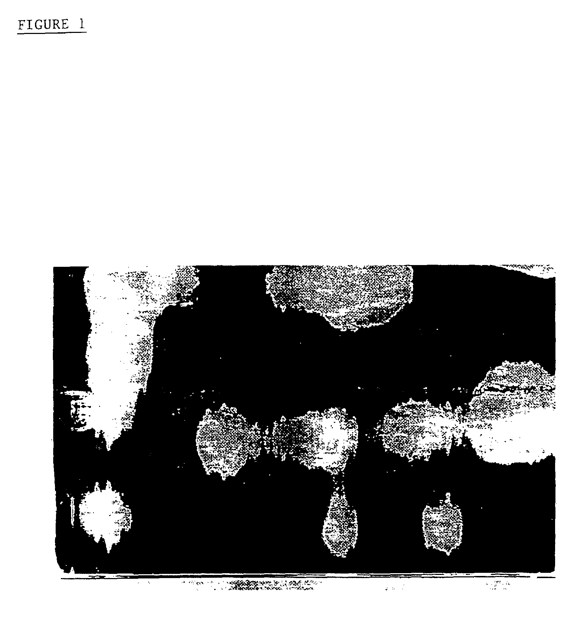

Figure 1 shows a gel prepared according to the method of the invention and imaged on a fluorescence imager (FluorImager, Molecular Dynamics). The image shows zones of reduced fluorescence (white) on a higher fluorescence background (dark), due to the inhibitor phosphoramidon diffusing from the beads and inhibiting the enzyme. The centre of each zone corresponds to a bead position.

### Example 1.

### Demonstration of polystyrene bead dispersal in a Sephacryl™ gel assay medium

Approximately 600 polstyrene beads (150-300 micron, derivatised with p-hydroxyphenoxymethyl groups, were suspended in Tetramethylrhodamine isothiocyanate (TRITC) in DMSO at ca 10mg/ml. The beads were allowed to swell. The beads were washed once briefly with DMSO and then added as a DMSO slurry (ca 0.1 ml) to 10 ml of Sephacryl™ S-400 (Pharmacia) as supplied and vortexed mixed thoroughly. This was poured into a polystyrene microtitre plate lid and allowed to stand for ca 30 minutes while excess water evaporated to give a solid gel. The beads were revealed as small purple foci which were evenly distributed throughout the gel. This shows that, despite the strong tendency of such polystyrene beads to float in aqueous media and aggregate, the beads may be successfully cast according to the method of the invention to give an even random distribution.

### Example 2.

### Demonstration of zones of enzyme inhibition in a gel

In this example, polystyrene beads are impregnated with a compound (phosphoramidon) to simulate the situation that would arise when a compound is made on a bead and then released prior to contacting the assay medium. The assay medium comprises a Sephacryl™ gel containing an enzyme, endothelin converting enzyme, and a fluorogenic FRET substrate, following by fluorescence imaging of the gel.

Ca 300 beads as used in Example 1 were suspended in a solution of phosphoramidon (10mM) in DMSO, allowed to swell and washed briefly in DMSO.

The FRET substrate, was derived from that of human big endothelin-1, 16-37, with Glu-26 changed to Cys: Fluorescein had been attached to Cys-26 and tetramethylrhodamine attached to the N-terminus, such that cleavage of the substrate removes the quenching effect of the tetramethylrhodamine resulting in an increase of fluorescein fluorescence.

Samples of endothelin converting enzyme (ECE) were obtained essentially according to the method of Okada et al., Biochem. Biophys. Res. Comm., 3, 1192-1198.

The assay phase was assembled as follows. Sephacryl™ S-500 gel was suspended in 0.2M Tris/HCl pH 7.2 containing 0.5% BSA and then washed with buffer alone. To this gel suspension (24.5ml) was added the FRET substrate (3ml, 4micromolar final) and ECE (2.6 microlitres). After gentle mixing, the gel was poured into the polystyrene lid of a microtitre plate, allowed to settle and excess liquid gently removed with a tissue. Beads were placed onto the surface of the gel using watchmakers forceps and the gel then left at ambient temperature overnight. The gel was then imaged on a fluorescence imager (FluorImager, Molecular Dynamics). The image, shown in Figure 1, shows zones of reduced fluorescence (white) on a higher fluorescence background (dark), due to the inhibitor phosphoramidon diffusing from the beads and inhibiting the enzyme. The centre of each zone corresponded to a bead position.

### Example 3.

### Detection of endothelin antagonists by zone screening employing scintillation proximity

Agarose (Sigma) was suspended in distilled water and heated to boiling point in a microwave to melt, then cooled to 50 deg. C. Microspheres coated with the porcine endothelin receptor (1 vial, SPA, Amersham) were re-constituted in assay buffer (50 mM Tris.HCl, 1 mM EDTA, 0.1% BSA, 10 mM MgC12, pH 6.4 (25 ml). This suspension (10 ml) was then added to the cooled (50 deg. C) agarose (10 ml), vortexed mixed and 0.1 ml portions immediately added to wells of a white microtitre plate (Picoplate-96, Packard).

Test compounds were prepared as follows. Polystyrene beads (as used in example 1) were suspended in DMSO solutions of endothelin-1 (Cambridge Research Biochemicals) or various test compounds (T1 - T4) at various concentrations (see Table 1) for about 2 hours. After a spin (1 min.) on a micro-fuge, the beads were washed briefly in ethanol. Single beads were then transfered with forceps into the centre of the wells containing the SPA/agarose mix, one bead per well.

[125-I]Endothelin (Amersham) was diluted into the assay buffer to give a 180 pM solution. 6 ml of this was added to 6 ml of 1% agarose (at 50 deg. C) and vortexed mixed. 0.05 ml aliquots were then immediately added to each well. The plate was then sealed (Topseal-S, Packard) and counted at intervals (Top Count, Packard) for 2 minutes per well. By reference to a total control (no compound present) and non-specific binding control (40 micromolar endothelin-1, measured after 8 hours incubation), the per cent inhibition caused by each compound as it diffused away from the bead was calculated.

The results are given in Table 1. This shows that the various compounds were able to interact with the bead-bound receptor and caused inhibition of the signal. This inhibition is related to the potency of the compound. Though no two-dimensional information was provided by this example, it clearly demonstrates that scintillation proximity assays can be carried out in a gel-format, according to the method of the invention.

**Table 1**

| **Inhibition of endothelin-1 and test compounds** | | | | | | | |
|---|---|---|---|---|---|---|---|
| a) based on readings at 8 hours: | | | | | | | |
| | | -------- inhibition (%) ------------ | | | | | |
| | | Conc.(micromolar): | | | | | |
| | IC-50 (nM) | 40 | 20 | 10 | 5 | 2.5 | 1.25 |
| endothelin-1 | 0.18 | (100) | 47.5 | 40.0 | 18.2 | 14.0 | 6.5 |
| T1 | 1.0 | 68.6 | 66.9 | 76.1 | 37.3 | 26.8 | 26.4 |
| T2 | 7.9 | 22.6 | 15.5 | 16.3 | 5.5 | -1.1 | -10.4 |
| T3 | 63 | 1.8 | 5.6 | 7.6 | 10.1 | 4.2 | 4.9 |
| T4 | 251 | -5.3 | -1.5 | -0.8 | -6.5 | -6.3 | -5.3 |

| b) based on readings at 2 hours: | | | | | | | |
|---|---|---|---|---|---|---|---|
| | | | -------- inhibition (%) ------------ | | | | |
| | | Conc. (micromolar) : | | | | | |
| | IC-50 (nM) | 40 | 20 | 10 | 5 | 2.5 | 1.25 |
| endothelin-1 | 0.18 | (100) | 85.2 | 36.3 | 19.5 | 12.3 | -0.6 |
| T1 | 1.0 | 74.8 | 72.5 | 79.7 | 47.3 | 36.2 | 32.9 |
| T2 | 7.9 | 19.6 | 2.1 | -9.7 | -19.5 | -23.0 | -41.6 |
| T3 | 63 | 9.2 | 7.9 | 11.8 | 18.4 | 14.3 | 16.3 |
| T4 | 251 | -8.9 | -5.0 | -7.2 | -11.7 | -10.6 | -11.7 |
| IC-50 values were determined by conventional filtration techniques (Holland J., et al., Anal. Biochem. (1994), 222, 516-518). | | | | | | | |

## Claims

1. A method for screening a compound library provided on solid phase, which method comprises releasing a proportion of the library from the solid phase into distinct zones of an assay medium, performing a proximity screening assay with a biological of interest within the medium, identifying active zone(s) in the assay medium and determining the identity of active member(s) of the library by reference to the corresponding compound(s) still bound to the solid phase.

2. A method as claimed in claim 1 wherein the compound library is a chemical compound library.

3. A method as claimed in claim 1 or claim 2 wherein the proximity screening assay is a fluorescent assay.

4. A method as claimed in any one of claims 1-3 wherein the proximity screening assay is a fluorescence resonance energy transfer (FRET) assay.

5. A method as claimed in claim 1 or claim 2 wherein the proximity screening assay is based on the proximity of a radiolabel to a scintillant.

6. A method as claimed in any one of the previous claims wherein the proximity screening assay is an immunoassay.

7. A method as claimed in claim 5 wherein the biological of interest and scintillant are bound to a solid phase.

8. A method for screening a chemical compound library provided on solid phase which method comprises releasing a proportion of the library from the solid phase into distinct zones of an assay medium and performing a screening assay with a biological of interest within the medium, identifying active zone(s) in the assay medium and determining the identity of active member(s) of the library by reference to the corresponding compound(s) still bound to the solid phase.

9. A method as claimed in claim 8 wherein the library is a combinatorial chemical compound library.

10. A method as claimed in any one of the previous claims wherein the solid phase comprises a plurality of individual solid supports.

11. A method as claimed in any one of the previous claims wherein the assay medium comprises a gel phase and the solid phase is immobilised by the gel phase.

12. A method as claimed in any one of the previous claims wherein the solid phase comprises a plurality of individual beads having a common visible tag.

13. A method as claimed in any one of the previous claims wherein the assay medium comprises two or more layers, each layer comprising one or more assay components.

14. A method as claimed in claim 13 wherein library compounds are released at or near the interface of two such layers.

15. A method as claimed in any one of the previous claims and which is repeated more than once using solid phase corresponding to active zone(s) from previous round(s).

## Patentansprüche

1. Verfahren zum Absuchen einer auf einer Festphase bereitgestellten Bank von Verbindungen, umfassend die Freisatzung eines Teils der Bank von der Festphase in getrennte Zonen eines Testmediums, Durchführung eines auf der Nähe der Komponenten basierenden Absuchtests ("proximity screening assay") mit einer biologischen Verbindung von Interesse in dem Medium, Identifizierung einer (von) aktiven Zone(n) im Testmedium und Bestimmung der Identität eines aktiven (aktiver) Mitglieds(Mitglieder) der Bank durch Bezug auf die entsprechende(n) Verbindung(en), die noch an die Festphase gebunden ist (sind).

2. Verfahren nach Anspruch 1, wobei die Verbindungsbank eine Bank chemischer Verbindungen ist.

3. Verfahren nach Anspruch 1 oder 2, wobei der auf der Nähe der Komponenten basierende Absuchtest ein Fluoreszenztest ist.

4. Verfahren nach einem der Ansprüche 1 bis 3, wobei der auf der Nähe der Komponenten basierende Absuchtest ein Fluoreszenzresonanzenergietransfer (FRET)-Test ist.

5. Verfahren nach Anspruch 1 oder 2, wobei der auf der Nähe der Komponenten basierende Absuchtest auf der Nähe einer Radiomarkierung zu einem Scintillator beruht.

6. Verfahren nach einem der vorangehenden Ansprüche, wobei der auf der Nähe der Komponenten basierende Absuchtest ein Immuntest ist.

7. Verfahren nach Anspruch 5, wobei die biologische Verbindung von Interesse und der Scintillator an die Festphase gebunden sind.

8. Verfahren zum Absuchen einer auf einer Festphase bereitgestellten Bank chemischer Verbindungen, umfassend die Freisetzung eines Teiles der Bank von der Festphase in bestimmte Zonen eines Testmediums und Durchführung eines Absuchtests mit einer biologischen Verbindung von Interesse im Medium, Identifizierung einer (von) aktiven Zone(n) in dem Testmedium und Bestimmung der Identität eines aktiven (aktiver) Mitglieds (Mitglieder) der Bank durch Bezug auf die entsprechende(n) Verbindung(en), die noch an die Festphase gebunden ist (sind).

9. Verfahren nach Anspruch 8, wobei die Bank eine kombinatorische Bank chemischer Verbindungen ist.

10. Verfahren nach einem der vorangehenden Ansprüche, wobei die Festphase eine Vielzahl von individuellen festen Trägern umfaßt.

11. Verfahren nach einem der vorangehenden Ansprüche, wobei das Testmedium eine Gelphase umfaßt und die Festphase durch die Gelphase immobilisiert ist.

12. Verfahren nach einem der vorangehenden Ansprüche, wobei die Festphase eine Vielzahl von individuellen Kügelchen mit einer üblichen sichtbaren Markierung umfaßt.

13. Verfahren nach einem der vorangehenden Ansprüche, wobei das Testmedium zwei oder mehr Schichten umfaßt, wobei jede Schicht eine oder mehrere Testkomponenten umfaßt.

14. Verfahren nach Anspruch 13, wobei die Bankverbindungen an oder nahe der Grenzfläche zweier solcher Schichten freigesetzt werden.

15. Verfahren nach einem der vorangehenden Ansprüche, welches mehr als einmal unter Verwendung einer Festphase, die der (den) aktiven Zone(n) vorstehender Runden entspricht, wiederholt wird.

## Revendications

1. Procédé pour cribler une banque de composés disposée sur une phase solide, lequel procédé comprend la libération d'une proportion de la banque depuis la phase solide dans des zones distinctes d'un milieu d'analyse, la mise en oeuvre d'une analyse de criblage de proximité avec une substance biologique d'intérêt dans le milieu, l'identification d'une ou plusieurs zones actives dans le milieu d'analyse et la détermination de l'identité d'un ou plusieurs membres actifs de la banque par référence au(x) composé(s) correspondant(s) encore lié(s) à la phase solide.

2. Procédé selon la revendication 1, dans lequel la banque de composés est une banque de composés chimiques.

3. Procédé selon la revendication 1 ou la revendication 2, dans lequel l'analyse de criblage de proximité est une analyse fluorescente.

4. Procédé selon l'une quelconque des revendications 1 à 3, dans lequel l'analyse de criblage de proximité est une analyse de transfert d'énergie de résonance de fluorescence (FRET).

5. Procédé selon la revendication 1 ou la revendication 2, dans lequel l'analyse de criblage de proximité est basée sur la proximité d'un radiomarqueur et d'un scintillateur.

6. Procédé selon l'une quelconque des revendications précédentes dans lequel l'analyse de criblage de proximité est une immunoanalyse.

7. Procédé selon la revendication 5, dans lequel la substance biologique d'intérêt et le scintillateur sont liés à la phase solide.

8. Procédé pour cribler une banque de composés chimiques disposée sur une phase solide, lequel procédé comprend la libération d'une proportion de la banque depuis la phase solide dans des zones distinctes d'un milieu d'analyse et la mise en oeuvre d'une analyse de criblage avec une substance biologique d'intérêt dans le milieu, identification d'une ou plusieurs zones actives dans le milieu d'analyse et la détermination de l'identité d'un ou plusieurs membres actifs de la banque par référence au(x) composé(s) correspondant(s) encore lié(s) à la phase solide.

9. Procédé selon la revendication 8, dans lequel la banque est une banque de composés chimiques combinatoire.

10. Procédé selon l'une quelconque des revendications précédentes, dans lequel la phase solide comprend une pluralité de supports solides individuels.

11. Procédé selon l'une quelconque des revendications précédentes, dans lequel le milieu d'analyse comprend une phase de gel et la phase solide est immobilisée par la phase de gel.

12. Procédé selon l'une quelconque des revendications précédentes, dans lequel la phase solide comprend une pluralité de billes individuelles ayant une marque visible commune.

13. Procédé selon l'une quelconque des revendications précédentes, dans lequel le milieu d'analyse comprend deux ou plusieurs couches, chaque couche comprenant un ou plusieurs composants d'analyse.

14. Procédé selon la revendication 13, dans lequel des composés de la banque sont libérés à l'interface ou à proximité de l'interface de deux telles couches.

15. Procédé selon l'une quelconque des revendications précédentes et qui est répété plus d'une fois à l'aide d'une phase solide correspondant à une ou des zones actives provenant d'une ou plusieurs opérations précédentes.


### SEQUENCE LISTING

### (1) GENERAL INFORMATION

(i) **APPLICANT:** ZENECA Limited
(ii) **TITLE OF INVENTION:** METHOD
(iii) **NUMBER OF SEQUENCES:** 1
(iv) CORRESPONDENCE ADDRESS:
   (A) ADDRESSEE: Intellectual Property Group, Zeneca Pharmaceuticals
   (B) STREET: Mereside, Alderley Park
   (C) CITY: Macclesfield
   (D) STATE: Cheshire
   (E) COUNTRY: United Kingdom
   (F) ZIP: GB-SK10 4TG
(v) **COMPUTER READABLE FORM:**
   (A) MEDIUM TYPE: DISKETTE, 3.5 INCH, 1.44 mB storage
   (B) COMPUTER: IBM PS/2
   (C) OPERATING SYSTEM: PC-DOS 5.2
   (D) SOFTWARE: ASCII from WPS-PLUS
(vi) **CURRENT APPLICATION DATA:**
   (A) APPLICATION NO:
   (B) FILING DATE:
(vii) **PRIOR APPLICATION DATA:**
   (A) APPLICATION NO: 9414770.9
   (B) FILING DATE: 22-Jul-94
(vii) **PRIOR APPLICATION DATA:**
   (A) APPLICATION NO: 9510137.4
   (B) FILING DATE: 19-May-95

### (2) INFORMATION FOR SEQ ID NO:1:

(i) SEQUENCE CHARACTERISTICS:
   (A) LENGTH: 22 amino acids
   (B) TYPE: amino acid
   (C) STRANDEDNESS: single
   (D) TOPOLOGY: linear
(xi) SEQUENCE DESCRIPTION: SEQ ID NO:1:
   His Leu Asp Ile Ile Trp Val Asn Thr Pro Cys His Val Val Pro Tyr
   1               5                   10                  15
   Gly Leu Gly Ser Pro Arg
               20